Europäisches Patentamt

European Patent Office

Office européen des brevets

(11) Veröffentlichungsnummer: **0 188 712**
**B1**

(12) **EUROPÄISCHE PATENTSCHRIFT**

(45) Veröffentlichungstag der Patentschrift:
31.01.90

(51) Int. Cl.⁴: **C 12 N 9/06**, C 12 N 1/20,
C 12 P 13/04, C 12 P 13/22 //
(C12N1/20, C12R1:01)

(21) Anmeldenummer: 85115344.5

(22) Anmeldetag: 03.12.85

(54) Verfahren zur Gewinnung von Phenylalanin-Dehydrogenase enthaltenden Mikroorganismen und deren Verwendung zur Herstellung von L-alpha-Aminosäuren.

(30) Priorität: 19.12.84 DE 3446304

(43) Veröffentlichungstag der Anmeldung:
30.07.86 Patentblatt 86/31

(45) Bekanntmachung des Hinweises auf die Patenterteilung:
31.01.90 Patentblatt 90/5

(84) Benannte Vertragsstaaten:
BE CH DE FR GB LI NL SE

(56) Entgegenhaltungen:
EP-A- 0 023 346
EP-A- 0 120 208
EP-A- 0 121 074
FR-A- 2 059 043
FR-A- 2 128 669
US-A- 3 036 958

INDUSTRIAL MICROBIOLOGY ABSTRACTS, Band 1, Nr. 2, 1965, Seite 5, Nr. 138; I. CHIBUTA et al.: "Production of L-phenylalanine from D-phenylalanine", & APPL. MICROBIOL. 1965, 13, 618
CHEMICAL ABSTRACTS, Band 97, Nr. 25, 20. Dezember 1982, Seite, Nr. 210708r, Columbus, Ohio, US; G. SAURET-IGNAZI et al.: "Generation of hydroen peroxide during the oxidation of L-phenylalanine by Proteus mirabilis isolated membranes", & BIOCHIMIE 1982, 64(10), 891-7

(73) Patentinhaber: Degussa Aktiengesellschaft,
Weissfrauenstrasse 9, D-6000 Frankfurt am Main 1 (DE)
Patentinhaber: Gesellschaft für Biotechnologische Forschung mbH (GBF), Mascheroder Weg 1,
D-3300 Braunschweig-Stöckheim (DE)

(72) Erfinder: Leuchtenberger, Wolfgang, Dr.,
Geschw.-Scholl-Strasse 1, D-6454 Bruchköbel (DE)
Erfinder: Kula, Maria-Regina, Dr., Forstweg 15,
D-3340 Wulfenbüttel (DE)
Erfinder: Hummel, Werner, Dr., Claudiusstrasse 11,
D-5117 Titz (DE)
Erfinder: Schütte, Horst, Nordring 29 A,
D-3320 Salzgitter 51 (DE)

(56) Entgegenhaltungen: (Fortsetzung)
AGRICULTURAL AND BIOLOGICAL CHEMISTRY, Band 47, Nr. 11, November 1983, Seiten 2511-2519, Tokyo, JP; N. ITOH et al.: "Crystallization and properties of L-alanine dehydrogenase from Streptomyces phaechromogenes"
CHEMICAL ABSTRACTS, Band 94, 11. Mai 1981, Seite 343, Nr. 153246a, Columbus, Ohio, US; O.N. SAKHNO et al.: "Capacity of Rhodopseudomonas sphaeroides for reduction amination", & BIOL. NAUKI 1981, (3), 84-7
CHEMICAL ABSTRACTS, Band 81, Nr. 23, 9. Dezember 1974, Seite 394, Nr. 150302m, Columbus, Ohio, US; & JP - A - 74 50 189 (SANRAKU OCEAN CO., LTD.) 25-09-1972
CHEMICAL ABSTRACTS, Band 57, 23. Juli 1962, Spalten 2679h-2680b, Columbus, Ohio, US; K. AIDA et al.: "Amino acid fermentation. VI. Preparation of L-tryptophan by bacterial reductive amination", & HAKKO TO TAISHA 1959, 1, 94-8

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Gewinnung von Phenylalanin-Dehydrogenase enthaltenden Mikroorganismen, Mikroorganismen und deren Verwendung zur Herstellung von L-α-Aminosäuren.

Für die stereospezifische Herstellung von L-Phenylalanin hat sich die Verwendung von Enzymen als Katalysatoren als vorteilhaft erwiesen. Aus der DE-OS 3 307 095 ist bekannt, Phenylpyruvat mit einer Phenylalanin-Dehydrogenase reduktiv zu aminieren und das entsprechende Enzym aus Brevibacterium spec. (DSM 2448) zu gewinnen. Das so isolierte Enzym erweist sich jedoch als relativ instabil.

Versuche, weitere die Phenylalanin-Dehydrogenase enthaltende Mikroorganismen mit Hilfe der dort beschriebenen Methode zu erhalten, führten nicht zum Erfolg.

Dies liegt darin begründet, dass das aus der DE-OS 3 307 095 bekannte Nährmedium ein unspezifisches Wachstum verschiedenster Mikroorganismen ermöglicht, was natürlich die Auswahl der gewünschten Stämme sehr erschwert.

Aufgabe der Erfindung ist ein Verfahren zur Isolierung von Phenylalanin-Dehydrogenase enthaltenden Mikroorganismen, das eine schnelle Auswahl geeigneter Stämme ermöglicht.

Gegenstand der Erfindung ist ein Verfahren zur Gewinnung von Phenylalanin-Dehydrogenase enthaltenden Mikroorganismen durch aerobes Kultivieren von Mikroorganismen-haltigen Proben in wässrigen Nährmedien, die eine Quelle für Kohlenstoff, Stickstoff und Mineralsalze und Thiamin enthalten, dadurch gekennzeichnet, dass man die Proben zuerst in einem Nährmedium mit einem Alkalipropionatgehalt von 0,1–0,5 g/l bebrütet, anschliessend entstandene Koloniemuster auf ein Nährmedium überimpft, das als Induktor für die Bildung von Phenylalanin-Dehydrogenase L-Phenylalanin oder L-Phenylalaninamid enthält, bebrütet und die Mikroorganismen auswählt, die in beiden Nährmedien wachsen.

Als Mikroorganismen-haltige Proben wählt man beispielsweise Bodenproben, die man mit steriler Salzlösung (0,9% NaCl) aufschlemmt, in üblicher Weise verdünnt und Aliquots auf Petrischalen mit festem Nährboden ausspatelt. Der Nährboden hat zum Beispiel die Standard-Zusammensetzung, der erfindungsgemäss ein Alkalipropionat, bevorzugt Natriumpropionat, hinzugesetzt wird:

| | |
|---|---|
| Propionsäure, Na-Salz | 0,1–0,5 g, bevorzugt 0,2 g |
| $KNO_3$ | 0,01 g |
| $KH_2PO_4$ | 0,1 g |
| NaCl | 0,25 g |
| $MgSO_4 \cdot 7H_2O$ | 100 mg |
| $CaCO_3$ | 20 mg |
| Spurenelemente-Lösung | 1 ml |
| Destilliertes Wasser | 1 l |
| Agar | 20 g |
| pH-Wert | 6,5–7,5, bevorzugt 7,0 |

Das Medium wird durch Autoklavieren sterilisiert, nach dem Abkühlen werden 1 ml sterilfiltrierte Vitaminlösung pro 1 l Medium sowie 50 mg Cycloheximid (Actidione) pro 1 l zugesetzt (Zusammensetzung der Vitaminlösung nach: Schlegel, H.G. «Allgemeine Mikrobiologie», Thieme Verlag, S. 169 [1981]) und der Nährboden in sterile Petrischalen gegossen.

Die beimpften Platten werden 4–6 Tage bei 23 bis 32°C, bevorzugt bei 27°C, bebrütet. Nach Wachstum der Mikroorganismen wird das Koloniemuster von Agarplatten mit einer geeigneten Koloniezahl (50–200) steril auf einen Samtstempel abgeimpft; dann auf 2 Platten überstempelt, die unterschiedliche Agarnährböden enthalten: Eine Platte enthält das oben angeführte Propionat-Medium, die andere ein Nährmedium mit L-Phenylalanin als Induktor und mit beispielsweise folgender Zusammensetzung (Phenylalanin-Medium):

| | |
|---|---|
| L-Phenylalanin | 1 bis 15 g, bevorzugt 10 g/l |
| $KH_2PO_4$ | 2 g |
| Hefeextrakt | 0,2 g |
| Agar | 20 g |
| Destilliertes Wasser | 1 l |
| pH-Wert | 6 bis 8, bevorzugt 7,2 |

Die Platten werden erneut 3–5 Tage bei 23 bis 32°C, bevorzugt bei 27°C, bebrütet. Durch Vergleich des Koloniemusters kann man diejenigen Organismen heraussuchen, die sowohl auf Propionat als auch auf Phenylalanin-Medium wachsen können; diese werden abgeimpft und im folgenden auf Phenylalanin-Medium gehalten.

Die isolierten Mikroorganismen werden dann in üblicher Weise auf Reinheit überprüft (Verdünnungsausstrich, Mikroskopieren), einheitlich erscheinende Stämme werden sodann bei 23 bis 32°C, bevorzugt bei 27°C vermehrt. Das Flüssigmedium hat folgende Zusammensetzung (MP-Medium):

| | |
|---|---|
| L-Phenylalanin | 1 bis 15 g, bevorzugt 10 g |
| Maisquellwasser (Trockenpulver) | 2 g |
| $KH_2PO_4$ | 2 g |
| Destilliertes Wasser | 1 l |
| pH-Wert | 6 bis 8, bevorzugt 7,2 |

Nach 2 bis 3 Tagen werden die Zellen sedimentiert und das Enzym aus den aufgeschlossenen Mikroorganismen in bekannter Weise gewonnen.

Mit dieser Isolierungsmethode ist es auf einfache Weise möglich, aus der Mikroorganismen-Population einer Bodenprobe diejenigen Organismen anzureichern und zu isolieren, die die gewünschte Phenylalanin-Dehydrogenase-Aktivität enthalten. So konnten aus 20 Bodenproben aus der Umgebung von Braunschweig 7 Isolate erhalten werden, die Phe-DH-Aktivität besitzen (Tab. 1). Die isolierten Organismen können in 2 Gruppen eingeteilt werden: 5 Isolate, die zur Gattung Brevibacterium gehören (jedes aus einer anderen Bodenprobe) sowie 2 Isolate, die zur Gattung Rhodococcus gehören, wie die folgenden Eigenschaften zeigen:

Wachstum in kurzen, gram-positiven Stäbchen, die mit zunehmendem Alter in kokkoide Formen übergehen; Zellen sind unbeweglich und bilden keine Sporen; strikt aerobes Wachstum; Säurebildung aus Glucose; Katalase und Nitratreduktion positiv, Harnstoffspaltung positiv; Gelatine-, Casein- und Stärkeabbau negativ; $H_2S$-Bildung negativ; Wachstum bei 41 °C negativ. Die Zellwände enthalten meso-Diaminopimelinsäure, als Zellwandzucker wurden Arabinose und Galaktose nachgewiesen. Die Menachinone sind vom Typ MK-8 (H2). Beide Isolate enthalten Tuberculostearinsäure und Mykolsäuren mit 30 bis 50 Kohlenstoffatomen. Die durch pyrolytische Spaltung aus Mycolsäuremethylestern freigesetzten Fettsäuren enthalten 12–18 Kohlenstoffatome. Die Koloniefarbe beider Isolate ist safrangelb (Typ 41 nach dem RAL-Farbcode) (Seiler, H., J. Gen. Microbiol. 129, 1433–1471 [1983]). Die Organismen benötigen Thiamin zum Wachstum.

Die Isolate sind ein weiterer Gegenstand der Erfindung und wurden am 7.9.84 bei der DSM hinterlegt, und zwar als Rhodococcus spec. M4 unter der Nr. 3041 und als Rhodococcus spec. 13 unter der Nr. 3040. Die Mikroorganismen können aufbewahrt werden als lyophylisierte Kultur, durch Einfrieren bei −80°C oder in flüssigem Stickstoff bei −196°C; Arbeitskulturen werden auf Schrägagar-Röhrchen (Phenylalanin-Medium) gehalten.

Beide Stämme verwerten die Aminosäure D-Phenylalanin nicht.

Tabelle 1: Charakterisierung der Isolate

| Bezeichnung des Isolats | Herkunft der Bodenprobe | Zugehörigkeit des Isolats | PheDH spez. Akt. (U/mg) | Volumenakt. (U/l) | Stabilität (% Restaktivität nach 4 Tagen 4°C) |
|---|---|---|---|---|---|
| M4 | Schlamm vom Ufer «Ententeich» | Rhodococcus | 16 | 13000 | 90% |
| 13 | Maisfeld Ribbesbüttel | Rhodococcus | 11 | 8600 | 44% |
| 12 | Maisfeld Ribbesbüttel | Brevibacterium | 1,5 | 640 | 0% |
| IV/6 | Rasen Eingang GBF | Brevibacterium | 1,0 | 520 | 0% |
| V/II | Komposterde | Brevibacterium | 0,5 | 205 | 0% |
| VI/4 | Rasen, Stöckheim (Matthiesen) | Brevibacterium | 0,1 | 133 | 0% |
| T2 | Parkplatz, Zimmerstrasse | Brevibacterium | 0,7 | 840 | 0% |

Vorversuche mit Rohextrakt-Enzympräparaten haben gezeigt, dass die Phenylalanin-Dehydrogenasen aus den einzelnen Isolaten der Gattung Brevibacterium vergleichbar sind mit dem in der DE-OS 3 307 095.4 beschriebenen Enzym in bezug auf die spezifische Enzymaktivität, Volumenausbeute, Induzierbarkeit und Stabilität.

Die Enzyme aus den beiden Isolaten der Gattung Rhodococcus erweisen sich im Vergleich dazu als deutlich überlegen:

a) Die Enzyme im Rohextrakt zeigen höhere spezifische Aktivitäten (20–30 U/mg Protein statt 2–5 U/mg);

b) man erreicht bei Wachstum auf 1% L-Phenylalanin höhere Volumenausbeuten (20–25000 U/l statt 3–6000 U/l);

c) die Enzyme sind von deutlich besserer Stabilität.

Die Phenylalanin-Dehydrogenase kann enzymologisch wie folgt beschrieben werden:

a) sie katalysiert die reduktive Aminierung von aromatischen α-Ketosäuren, der systematische

Name ist L-Phenylalanin: $NAD^+$-oxidoreduktase (desaminierend), und sie gehört in die E.C.-Gruppe 1.4.1;

b) optimaler pH-Wert der reduktiven Aminierung von Phenylpyruvat ist 9,3± 1;

c) optimaler pH-Wert der oxidativen Desaminierung von L-Phenylalanin ist 10,0± 1;

d) der $K_M$-Wert für NADH beläuft sich auf 0,08 mM.

L-Phenylalanin und L-Phenylalaninamid werden bevorzugt als Induktoren eingesetzt, aber auch L-Histidin ist in begrenztem Umfang geeignet.

Die Phenylalanin-Dehydrogenase wird eingesetzt, um in Gegenwart von Ammoniumionen und NADH als Coenzym Phenylpyruvat, p-Hydroxyphenylpyruvat, Indolpyruvat oder 2-Keto-4-methylmercaptobutyrat ... in die entsprechenden L-α-Aminocarbonsäuren umzuwandeln.

Das Verfahren wird bevorzugt bei 20 bis 30°C und einem pH-Wert von 8,5 bis 10 durchgeführt. Vorteilhaft arbeitet man unter Coenzym-Regenerierung, wie sie aus DE-OS 3 307 095 bekannt ist.

In analoger Weise kann man auch nicht aufgeschlossene Bakterienzellen verwenden.

Das Enzym ist auch geeignet, auf enzymatischem Wege in wässrigen Lösungen die Konzentration der oben erwähnten α-Ketocarbonsäuren bzw. α-Aminosäuren zu bestimmen.

Nach Identifizierung der Isolate wurden bekannte Stämme der Gattung Rhodococcus aus der Stammsammlung (Deutsche Sammlung von Mikroorganismen, Göttingen) auf Phenylalanin-Dehydrogenase-Aktivität getestet. Zur Zeit sind 11 Arten dieser Gattung bekannt (Goodfellow, M. und Minnikin, D.E. in: The Prokaryotes (Starr, M.P. et al. eds.) S. 2016–2027, Springer Verlag, Berlin, Heidelberg, New York, (1981); Goodfellow, M., System. Appl. Microbiol. 5, 225–229 (1984)); jeweils der Typstamm wurde in MP-Medium (1% L-Phenylalanin, 0,2% Maisquellwasser, 0,2% $KH_2PO_4$) angezogen. Nach 40 Stunden Wachstum bei 27°C wurden die Zellen aufgeschlossen und im Rohextrakt die Aktivität der Phenylalanin-Dehydrogenase bestimmt.

Es zeigte sich, dass nur die Stämme von Rhodococcus erythropolis (DSM 43 066 und 743) L-Phenylalanin verwerten ($OD_{578}$ 28,0 für 43 066 und OD 15,3 für 743) und Phenylalanin-Dehydrogenase Aktivitäten besitzen (1260 U/l für 43 066 und 1031 U/l für 743), die aber deutlich unter denen der neuen Rhodococcusstämme liegen.

Grössere Mengen an Phenylalanin-Dehydrogenase kann man aus Rhodococcus spec. M4 und I3 gewinnen, indem man die Mikroorganismen in bekannter Weise in einem Bioreaktor in gewünschtem Massstab anzieht und daraus das Enzym isoliert.

Für eine erfolgreiche Anzucht gelten folgende bevorzugte Bedingungen:

a) eine gute Belüftung (obligat aerober Organismus);
b) ein Ausgangs-pH-Wert von 6,0–8,0; bevorzugt 7,0–7,6;
c) die Anwesenheit von Mineralsalzen (z.B. in komplexer Form als Maisquellwasser);
d) die Anwesenheit von geringen Mengen an Thiamin (1–2 μg/l);
e) ein L-Phenylalanin-Gehalt von 0,5–1,5% im Medium zur Induktion der PheDH;
f) eine Temperatur von 23 bis 32°C.

In den Proben werden gemessen:
a) die optische Dichte (Trübung) der Kultur bei 578 nm als Mass für das Wachstum;
b) der Phenylalaningehalt im Medium (nach Abzentrifugieren der Zellen) mit Hilfe eines Aminosäureanalysators;
c) der Enzymgehalt unter Anwendung eines photometrischen Tests. Der Testansatz enthält: 0,7 M Ammoniumchlorid/Ammoniakpuffer pH 8.5, 0.1 mM NADH, 10 mM Phenylpyruvat und limitierende Mengen an Enzym (1–20 μg Protein pro Test). Die Abnahme der Extinktion von NADH bei 340 nm wird gemessen. Von den erhaltenen Werten wird ein Nullwert abgezogen, dem man erhält, wenn der Test ohne Zugabe von Phenylpyruvat abläuft. Die Enzymaktivität wird in internationalen Einheiten angegeben, wobei eine Einheit die Abnahme von 1 μMol NADH/min bedeutet.

Für einen technischen Einsatz können die durch Züchtung erhaltenen Zellen direkt verwendet werden oder das in den Zellen enthaltene Enzym isoliert und dann im zellfreien System eingesetzt werden. Die Enzymisolierung ist nach Zellaufschluss durch Kombination an sich bekannter Methoden der Enzymreinigung möglich.

Zuerst setzt man aus der Bakterien-Feuchtmasse, bevorzugt durch einen mechanischen Zellaufschluss, zum Beispiel mit Hilfe einer Glasperlenmühle, eines Hochdruckhomogenisators oder einer Ultraschallbehandlung, die Inhaltsstoffe frei.

In einem zweiten Schritt werden die Zellbruchstücke gemäss DE-PS 2 639 129 aus dem Rohextrakt über ein wässriges Zweiphasensystem abgetrennt.

Die Oberphase enthält praktisch die gesamte Aktivität an L-Phenylalanin-Dehydrogenase und wird weiter einem Dialyseverfahren unterworfen.

Das dialysierte Enzym wird anschliessend mit Hilfe der DEAE-Cellulose-Chromatographie weitergereinigt und erreicht dann den gewünschten Grad der Aufreinigung nach Durchlaufen der Interfacial Salting Out-Chromatographie, zum Beispiel an Sepharose 4B.

Beispiel 1

Produktion der Phenylalanin-Dehydrogenase Zur Anzucht wird ein 2 l Bioreaktor verwendet, der mit 1,5 l Medium gefüllt ist. Das Medium enthält pro l 10 g L-Phenylalanin; 2 g $KH_2PO_4$; 2 g Maisquellwasser (Trockenpulver); der pH-Wert beträgt 7,0.

Nach Sterilisation wird das Medium mit 50 ml einer Vorkultur angeimpft, die 48 Stunden im gleichen Medium angezogen wurde. Die Wachstumsbedingungen im Fermenter sind:

Temperatur: 27°C
Belüftungsrate: 60 l Luft/Std.
Turbinenrührer mit 400 UpM

Zu verschiedenen Zeiten des Wachstums wurden Proben (40 ml) entnommen und nach einem Test auf enzymatische Aktivität in diesen Zellen der maximal erreichbare Enzymgehalt bzw. der günstigste Erntezeitpunkt bestimmt.

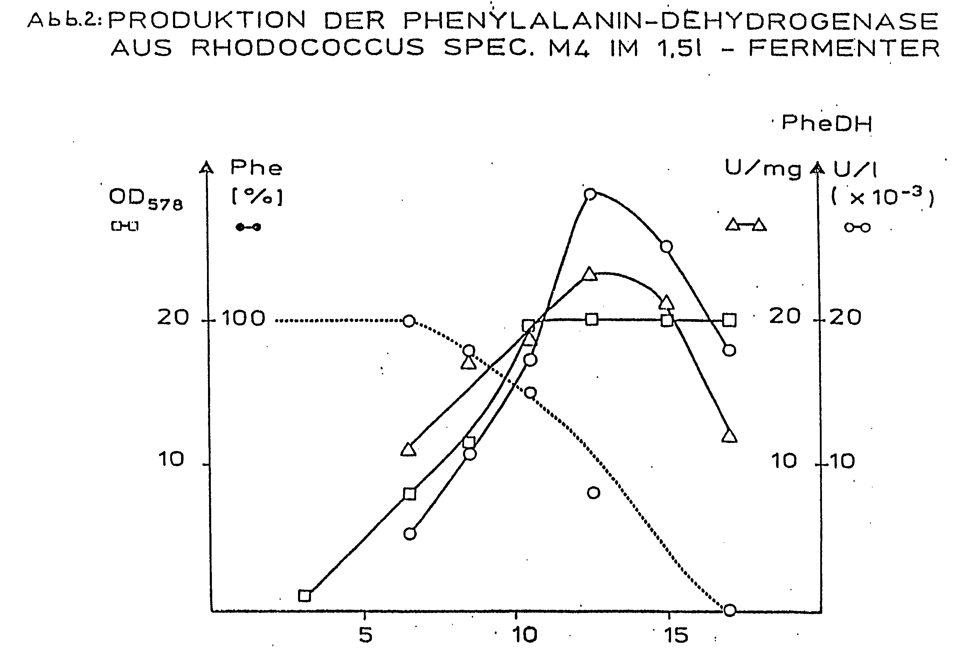

Die Abb. 2 zeigt, dass das Enzym in einer frühen Wachstumsphase gebildet wird; der Enzymgehalt steigt besonders an, wenn der Organismus anfängt, Phenylalanin abzubauen. Enzymgehalte von deutlich über 20000 Einheiten pro 1 Liter Medium können hier erreicht werden. Im weiteren Verlauf des Wachstums sinkt der Enzymgehalt dann wieder ab.

Aus einer 1,5 l Fermentation wurden nach Abzentrifugieren der Bakterien 70 g Zellen (Feuchtmasse) mit einer Gesamt-Enzymaktivität von 22000 Einheiten erhalten.

Beispiel 2
Bildung der PheDH in Abhängigkeit von der Aminosäure im Medium

Stamm M4 wird in einem Medium angezogen mit 0,2% Maisquellwasser (Trockenpulver), 0,2% $KH_2PO_4$ und jeweils 1% einer Aminosäure; der pH-Wert beträgt vor Beginn der Kultivierung 7,4. Es wurden jeweils 100 ml Medium beimpft; nach 40 Std. Wachstum die Zellen abzentrifugiert und nach Aufschluss der Zellen mit Glasperlen die Aktivität der PheDH bestimmt. Tabelle 2 zeigt, dass für eine gute Enzymausbeute der Zusatz von L-Phenylalanin notwendig ist, andere natürliche Aminosäuren ergeben geringere (Histidin) oder eine (Isoleucin) Enzymgehalte. L-Phenylalanin kann als Induktor durch das Derivat L-Phenylalanin-Amid ersetzt werden. Auf einigen natürlichen Aminosäuren kann der Stamm M4 nicht wachsen (z.B. L-Tryptophan, L-Tyrosin, L-Alanin oder L-Glutaminsäure). Ebenso verwertet der Stamm im Gegensatz zu Brevibacterium spec. (DSM 2448) die Aminosäure D-Phenylalanin nicht.

Tabelle 2

| Aminosäure | $OD_{578}$ | U/mg | U/l |
|---|---|---|---|
| L-Phenylalanin | 31 | 7,25 | 15 200 |
| L-Histidin | 4 | 1,81 | 820 |
| L-Phenylalanin-Amid | 8 | 8,49 | 3550 |
| L-Isoleucin | 14 | 0 | 0 |
| D-Phenylalanin | 0 | | |

Beispiel 3
Bildung der PheDH in Abhängigkeit von der Phenylalanin-Konzentration im Medium

Stamm M4 wird in einem Medium angezogen mit 0,2% Maisquellwasser (Trockenpulver), 0,2% $KH_2PO_4$ und steigenden Mengen L-Phenylalanin (bis 1,5%); der pH-Wert vor Beginn der Kultivierung beträgt 7,4.

Es wurden jeweils 100 ml Medium beimpft; nach 40 Stunden Wachstum wurden die Zellen abzentrifugiert und nach Aufschluss der Zellen mit Glasperlen die Aktivität der PheDH bestimmt. Die Tabelle 3 zeigt, dass durch Erhöhung des Phenylalanin-Gehalts im Medium auch die Enzymausbeute erhöht werden kann, mit 1,5% Phenylalanin erhält man so ca. 26 000 U pro 1 l Medium. Der Verlauf der spezifischen Enzymaktivität (U/mg) zeigt zudem, dass ein Gehalt an Phenylalanin über 0,5% zu bevorzugen ist.

Tabelle 3

| L-Phe g/l | Phenylalanin-Dehydrogenase | |
|---|---|---|
| | U/mg | U/l |
| 1 | 0 | 0 |
| 2 | 0 | 0 |
| 5 | 1 | 2000 |
| 8 | 12,2 | 14000 |
| 10 | 14,6 | 18000 |
| 12 | 16,6 | 23000 |
| 15 | 20,4 | 26000 |

Beispiel 4
Abhängigkeit der Reaktionsgeschwindigkeit vom pH-Wert

a) Reduktive Aminierung
Die Reaktionsgeschwindigkeit der reduktiven Aminierung von Phenylpyruvat zu L-Phenylalanin wurde in Abhängigkeit vom pH-Wert der Reaktionslösung untersucht. Der Testansatz hatte folgende Zusammensetzung:

0,25 mM NADH, 1,5 mM Phenylpyruvat und limitierende Mengen an Enzym in einer 0,7 M Ammoniumchloridlösung bei unterschiedlichen pH-Werten. Unterschiedliche pH-Werte wurden durch Zugabe von Ammoniak bzw. Salzsäure zu der 0,7 M Ammoniumchloridlösung vor Zusammenmischen des Testansatzes eingestellt.

In Abb. 2 ist die Reaktionsgeschwindigkeit als Funktion des pH-Wertes im Bereich von 6,0 bis 10,2 aufgetragen. Das pH-Optimum liegt bei 9,25. Der pH-Wert wurde im Reaktionsgemisch gemessen.

b) Oxidative Desaminierung
Die Reaktionsgeschwindigkeit der oxidativen Desaminierung von L-Phenylalanin, katalysiert durch die L-Phenylalanin-Dehydrogenase, wird gleichfalls in Abhängigkeit vom pH-Wert untersucht. Der Testsansatz hatte folgende Zusammensetzung:

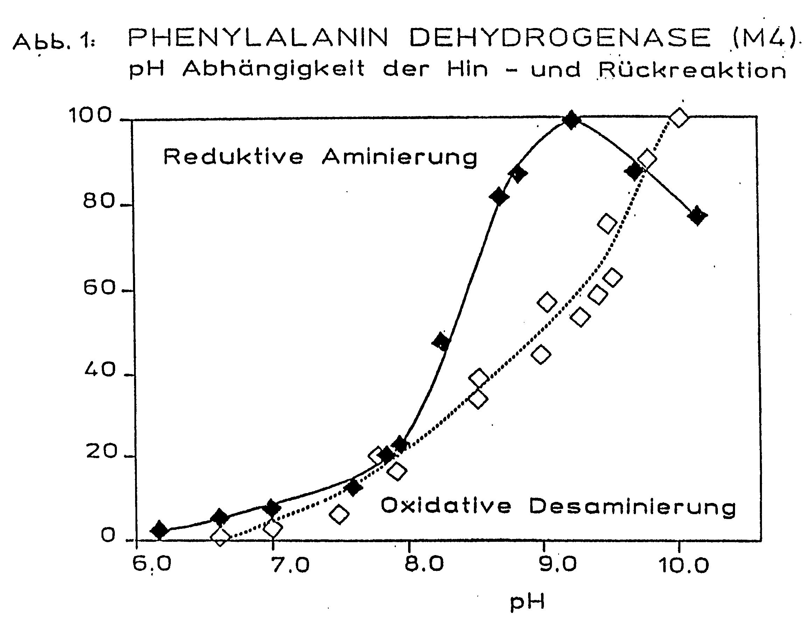

3 mM $NAD^+$ und 6 mM L-Phenylalanin in einem 0,1 M Glycin/NaCl/NaOH-Puffer bei unterschiedlichen pH-Werten. Es wurden unterschiedliche pH-Werte durch Zugabe von Salzsäure oder Natronlauge zu dem Puffer vor Zusammenmischen des Testsansatzes eingestellt. Die Ergebnisse sind zusammenfassend gleichfalls in Abb. 1 dargestellt für den Bereich von pH 6,5 bis 10,1. Die Rückreaktion steigt bis pH 10,1 an. Der pH-Wert wurde im Reaktionsgemisch gemessen.

Beispiel 5
Abhängigkeit der Reaktionsgeschwindigkeit von den Substratkonzentrationen

Reduktive Aminierung
a) Die Abhängigkeit der Reaktionsgeschwindigkeit der reduktiven Aminierung von Phenylpyruvat zu L-Phenylalanin für das Substrat NADH wurde in folgendem Testansatz untersucht:

0,7 M Ammoniumchlorid/Ammoniak-Puffer (pH 9,5), 1,5 mM Phenylpyruvat, limitierende Mengen an Enzym. Die NADH-Konzentration im Testansatz wurde im Bereich von 0,025-0,3 mM variiert.

Es zeigte sich, dass die optimale Reaktionsgeschwindigkeit bei 0,25 mM erreicht wird. Der $K_M$-Wert für NADH beträgt 0,08 mM.

b) Die reduktive Aminierung verschiedener α-Ketocarbonsäuren wurde in Abhängigkeit von der Ketosäurekonzentration untersucht. Dazu wurde folgender Testansatz verwendet:

0,7 M Ammoniumchlorid/Ammoniak-Puffer (pH 9,5), 0,25 mM NADH, limitierende Mengen an Enzym. Die Ketosäurekonzentration wurde jeweils im Bereich von 0,01 bis 30 mM variiert.

Die Anfangsreaktionsgeschwindigkeit (Extinktionsänderung 340 nm/Minute) wird nach Michaelis-Menten ausgewertet. Die gefundenen $K_M$- und $V_{max}$-Werte sind in Tabelle 4 zusammengefasst. Wegen der Störung des optischen Tests im Falle der Substrate Indolpyruvat und p-Hydroxyphenylpyruvat wurden die reduktiven Aminierungen zu L-Tryptophan bzw. Tyrosin als Funktion der Zeit am Aminosäureanalysator bestimmt (Biotronik BC 6000, ausgerüstet mit einem Integrator Biotronik, System 1, in einem 1-Säulen-Programm; als Eichlösung wurde ein Aminosäure-Standard IV der Firma Pierce verwendet).

Oxidative Desaminierung

a) Die Abhängigkeit der Reaktionsgeschwindigkeit der oxidativen Desaminierung von L-Phenylalanin von der $NAD^+$-Konzentration wurde in folgendem Testansatz untersucht:

0,1 M Glycin-NaCl/NaOH-Puffer (pH 10,7, 4 mM L-Phenylalanin, limitierende Mengen an Enzym. Die $NAD^+$-Konzentration wurde im Bereich von 0,1 bis 5,0 mM variiert. Es zeigte sich, dass der optimale Umsatz bei einer Konzentration von 3 mM erreicht wird.

b) Die Abhängigkeit der Reaktionsgeschwindigkeit der oxidativen Desaminierung von der L-Aminosäure-Konzentration wurde in folgendem Testansatz untersucht:

0,1 M Glycin-NaCl/NaOH-Puffer (pH 10,7), 3 mM NAD, limitierende Mengen an Enzym. Die L-Aminosäure-Konzentration wurde im Bereich von 0,3 bis 15 mM variiert.

Der $K_M$-Wert für L-Phenylalanin beträgt 0,8 mM; die entsprechenden Werte für die anderen Aminosäuren sind in Tabelle 5 zusammengestellt.

Tabelle 4: Substratspezifität für die reduktive Aminierung der Phenylalanin-Dehydrogenase

| Substrat | $V_{max}$ (relativ zum Phenylpyruvat) | $K_M$ [M] |
|---|---|---|
| Phenylpyruvat | 100 | $1,6 \cdot 10^{-4}$ |
| p-Hydroxyphenyl-pyruvat | 5 | $2,4 \cdot 10^{-3}$ |
| Indolpyruvat | 3 | $7,7 \cdot 10^{-3}$ |
| 2-Keto-4-methyl-mercapto butyrat | 33 | $2,1 \cdot 10^{-3}$ |
| NADH | | $8 \cdot 10^{-5}$ |

Tabelle 5: Substratspezifität für die oxidative Desaminierung der Phenylalanin-Dehydrogenase

| Substrat | $V_{max}$ (relativ zu L-Phenylalanin) | $K_M$ M |
|---|---|---|
| L-Phenylalanin | 100 | $7,5 \cdot 10^{-4}$ |
| L-Tryptohan | 2 | $1,05 \cdot 10^{-2}$ |
| L-Methionin | 4 | $4,3 \cdot 10^{-3}$ |
| $NAD^+$ | | $2,2 \cdot 10^{-4}$ |

Beispiel 6

Stabilität der PheDH

Stamm M4 wird in 100 ml Medium folgender Zusammensetzung angezogen:

0,2% Maisquellwasser (Trockenpulver), 0,2% $KH_2PO_4$, 1% L-Phe, pH-Wert beträgt 7,0. Nach 40-stündiger Inkubation bei 27°C auf der Rundschüttelmaschine werden die Zellen durch Zentrifugation geerntet, mit Glasperlen aufgeschlossen und der Zellüberstand durch Zentrifugation des Zellbreis erhalten (Rohextrakt). In gleicher Weise wird Rohextrakt von Brevibacterium spec. (DSM 2448) gewonnen. In beiden Extrakten werden die PheDH-Aktivitäten bestimmt, dann werden Aliqots bei 4°C und bei 22°C gelagert und nach bestimmten Zeiten die Restaktivität bestimmt.

Tabelle 6 zeigt, dass die Lagerstabilität der PheDH aus Stamm M4 im Rohextrakt deutlich besser ist als die der PheDH aus Brevibacterium spec., nach 1 Woche bei 4°C sind noch 90% Aktivität nachweisbar.

Tabelle 6

| Enzym aus | Lager-tempe-ratur (°C) | Restaktivität nach Tagen 1 2 3 4 (%) | | | |
|---|---|---|---|---|---|
| Rhodococcus spec. | 4 | 98 | 93 | 93 | 91 |
| Rhodococcus spec. | 22 | 84 | 74 | 66 | 60 |
| Brevibacterium spec. | 4 | 70 | 55 | 30 | 20 |

Beispiel 7

Herstellung von L-Phenylalanin mit ganzen Zellen

Die stereospezifische Umsetzung von Phenylpyruvat wird auch erreicht bei Verwendung der ganzen Mikroorganismen-Zelle. Inkubiert man unter geeigneten Bedingungen Phenylpyruvat, Glucose und ein Ammoniumsalz mit Zellen von Stamm M4, kann die Bildung von Phenylalanin nachgewiesen werden, das für die Umsetzung notwendige NADH wird dabei durch die Enzyme des Glucosemetabolismus kontinuierlich regeneriert.

Im einzelnen enthielt der Ansatz:
2 ml $NH_4Cl$-Lösung pH 7,0 (200 mM)
3 ml Zellsuspension (0,1 g Feuchtmasse pro 1 ml)
2 ml Glucose (200 mM)
1 ml $KH_2PO_4$ pH 7,0 (0,1 M)
2 ml Phenylpyruvat (50 mM)

In Klammern sind die Endkonzentrationen der Einzelkomponenten im Test angegeben. Als Enzymquelle wurde eine Zellsuspension von Stamm M4 verwendet, die entsprechend Beispiel 1 gewonnen wurde.

Zum Vergleich wurde zudem ein paralleler Ansatz inkubiert, der Zellen von Brevibacterium spec. DSM 2448 enthält.

Die Inkubationen wurden in 100 ml Erlenmeyer-Kolben bei 30 °C auf einer Schüttelmaschine (100 UpM) durchgeführt. Zu verschiedenen Zeiten wurden Proben entnommen und der Gehalt an gebildetem Phenylalanin mit einem Amino-Säurereanalysator bestimmt.

Wie die Tabelle 7 zeigt, sind die Zellen von beiden Mikroorganismen in der Lage, Phenalalanin zu bilden: Stamm M4 hat nach 7 Stunden 36,1 uMol Phe/ml (= 53 mg Phe/g Bakterien-Feuchtmasse) gebildet; das entspricht einer Umsetzung von 64% des eingesetzten Substrats. Brevibacterium spec. (DSM 2448) hat 18,5 uMol Phe/ml gebildet (= 46,1 mg Phe/g Bakterien-Feuchtmasse); das entspricht einer Umsetzung von 37% des eingesetzten Phenylpyruvats.

| Stamm | Phenylalanin nach | | |
| | 1 h | 3 h | 7 h |
| | (mg/g Zellen) | | |
| --- | --- | --- | --- |
| Rhodococcus spec. | 23 | 35 | 53 |
| Brevibacterium spec. | 15,5 | 27,5 | 46 |

Beispiel 8

Herstellung von L-Phenylalanin im zellfreien System unter NADH-Regenerierung

Die stereospezifische Umsetzung von Phenylpyruvat zu L-Phenylalanin kann im zellfreien System mit der Phenylalanin-Dehydrogenase durchgeführt werden. Der Reaktionsgleichung entsprechend muss das Coenzym NADH zugesetzt werden. Um das bei der Umsetzung oxidierte Coenzym zu regenerieren, werden dem Ansatz Formiat-Dehydrogenase (E.C. 1.2.1.2) und Formiat zugesetzt, als zusätzliches Produkt erhält man dann $CO_2$.

Der Ansatz enthält im einzelnen:
400 mM Ammoniumformiat (pH 9,2)
150 mM Tris-HC1 (pH 9,2)
0,3 mM NADH
2 U/ml Formiat-Dehydrogenase (Präparat gemäss Kroner et al. (1982), J. Chem. Tech. Biotechnol. 32, 130–137)
2 U/ml Phenylalanin-Dehydrogenase (Präparat mit 110 U/mg; Top-Phase nach 1. Flüssig-Flüssig-Verteilung, gemäss Tab. 2
20 mM Phenylpyruvat

Das Gesamtvolumen beträgt 2 ml, die Inkubation erfolgte unter Rühren bei 28 °C. Die Produktbildung wurde am Aminosäure-Analysator verfolgt.

Die Tab. zeigt, dass nach 90 min 19 mM Produkt gebildet wurden (96% Umsatz).

Tab.: Umsetzung von Phenylpyruvat zu L-Phenylalanin im zellfreien System (Umsatz = bezogen auf eingesetztes Phenylpyruvat)

| Zeit min | L-Phe mM | Umsatz % |
| --- | --- | --- |
| 15 | 7,4 | 37 |
| 30 | 12,8 | 64 |
| 60 | 16,8 | 84 |
| 90 | 19,1 | 96 |

Patentansprüche

1. Verfahren zur Gewinnung von Phenylalanin-Dehydrogenase enthaltenden Mikroorganismen durch aerobes Kultivieren von Mikroorganismen-haltigen Proben in wässrigen Nährmedien, die eine Quelle für Kohlenstoff, Stickstoff und Mineralsalze und Thiamin enthalten, dadurch gekennzeichnet, dass man die Proben zuerst in einem Nährmedium mit einem Alkalipropionatgehalt von 0,1 bis 0,5 g/l bebrütet, anschliessend entstandene Koloniemuster auf ein Nährmedium überimpft, das als Induktor für die Bildung von Phenylalanin-Dehydrogenase L-Phenylalanin oder L-Phenylalaninamid enthält, bebrütet, und die Mikroorganismen auswählt, die in beiden Nährmedien wachsen.

2. Rhodoccus specM4, hinterlegt bei der Deutschen Sammlung für Mikroorganismen unter der Nr. DSM 3041.

3. Rhodoccus specl3, hinterlegt bei der Deutschen Sammlung für Mikroorganismen unter der Nr. DSM 3040.

4. Verfahren zur Herstellung von Phenylalanin-Dehydrogenase durch Kultivieren eines Mikroorganismus, dadurch gekennzeichnet, dass man als Mikroorganismus die Stämme Rhodoccus specM4 (DSM 3041) oder Rhodococcus specl3 (DSM 3040) in einem Medium kultiviert, das L-Phenylalanin oder L-Phenylalaninamid enthält.

5. Verwendung der Rhodoccusstämme gemäss den Ansprüchen 2 und 3 zur reduktiven Aminierung von Phenylpyruvat, p-hydroxyphenylpyruvat, Indolylpyruvat oder 2-Keto-4-(methylmercapto)buttersäure in die entsprechenden L-α-Aminosäuren.

Claims

1. Process for obtaining microorganisms containing phenylalanine dehydrogenase by aerobic culture of microorganism-containing samples in aqueous nutrient media which contain a source of carbon, nitrogen and mineral salts and thiamine, characterized in that the samples are first incubated in a nutrient medium with an alkali metal propionate content of 0.1 to 0.5 g/l, colony patterns formed are then transinoculated onto a nutrient medium which contains L-phenylalanine or L-phenylalanine amide as an inducer for the formation of phenylalanine dehydrogenase, the system is incubated and the microorganisms which grow in both nutrient media are selected.

2. Rhodococcus spec M4, deposited at the Deutsche Sammlung für Mikroorganismen under no. DSM 3041.

3. Rhodococcus spec I3, deposited at the Deutsche Sammlung für Mikroorganismen under no. DSM 3040.

4. Process for the preparation of phenylalanine dehydrogenase by culture of a microorganism, characterized in that the strains Rhodococcus spec M4 (DSM 3041) or Rhodococcus spec I3 (DSM 3040) are cultured in a medium containing L-phenylalanine or L-phenylalanine amide.

5. Use of the Rhodococcus strains according to claims 2 and 3 for reductive amination of phenyl pyruvate, p-hydroxyphenyl pyruvate, indolyl pyruvate or 2-keto-4-(methylmercapto)butyric acid into the corresponding L-α-amino acids.

**Revendications**

1. Procédé d'obtention de micro-organismes contenant de la phénylalanine-deshydrogénase par culture aérobie d'échantillons contenant ces microorganismes dans des milieux nutritifs aqueux contenant une source pour le carbone, l'azote et les sels minéraux et de la thiamine, caractérisé en ce que l'on incube en premier lieu l'échantillon dans un milieu nutritif ayant une teneur en propionate de métal alcalin de 0,1 à 0,5 g/l, ensuite on sur-inocule les échantillons de colonies qui se sont formées sur un milieu nutritif contenant en tant qu'inducteur pour la formation de phénylalanine-deshydrogénase, de la L-phénylalanine ou de la L-phénylalanine amide, met en incubation et sélectionne les micro-organismes qui poussent dans les deux milieux nutritifs.

2. Rhodococcus spec. M4 déposé à la collection allemande pour les micro-organismes sous le no DSM 3041.

3. Rhodococcus spec. I3 déposé à la collection allemande pour les micro-organismes sous le no DSM 3040.

4. Procédé d'obtention de phénylalanine-deshydrogénase par culture d'un micro-organisme, caractérisé en ce que l'on cultive comme micro-organisme les souches Rhodococcus sp. M4 (DSM 3041) ou Rhodococcus sp. I3 (DSM 3040) dans un milieu qui contient de la L-phénylalanine ou de la L-phénylalanine amide.

5. Utilisation des souches de Rhodococcus selon les revendications 2 et 3 pour l'amination réductrice des phénylpyruvate, p-hydroxypyruvate, indolylpyruvate, ou de l'acide 2-céto-4-(méthylmercapto)butyrique en L-α-amino-acide correspondant.

# Abb. 1: PHENYLALANIN DEHYDROGENASE (M4).
## pH Abhängigkeit der Hin – und Rückreaktion

Reduktive Aminierung

Oxidative Desaminierung

pH

EP 0 188 712 B1

**Abb.2: PRODUKTION DER PHENYLALANIN-DEHYDROGENASE AUS RHODOCOCCUS SPEC. M4 IM 1,5l – FERMENTER**

EP 0 188 712 B1